# EUROPÄISCHE PATENTANMELDUNG

(11) **EP 4 480 420 A1**
(43) Veröffentlichungstag der Anmeldung: **25.12.2024**
(21) Anmeldenummer: 24181899.6
(22) Anmeldetag: 13.06.2024
(51) Int. Cl.: A61B 6/04, A61B 6/50

(54) **VORRICHTUNG ZUR VETERINÄRMEDIZINISCHEN DIAGNOSTIK MIT EINEM CT-GERÄT**

(30) Priorität: 21.06.2023 AT 504862023
(71) Anmelder: Schöndorfer, Martin, 4076 St. Marienkirchen (AT)
(72) Erfinder: Schöndorfer, Martin, 4076 St. Marienkirchen (AT)
(74) Vertreter: KLIMENT & HENHAPEL

(57) **Zusammenfassung**

Vorrichtung zur veterinärmedizinischen Diagnostik mit einem eine Gantry (2) umfassenden Computertomographie (CT)-Gerät und einem die Öffnung der Gantry (2) querenden CT-Tisch. Es wird vorgeschlagen, dass eine eine ebene Aufstandsfläche definierende Plattform (1) vorgesehen ist, die mittels einer Hebe- und Senkvorrichtung mit ihrer Aufstandsfläche heb- und senkbar ist, wobei der CT-Tisch (3) auf der Plattform (1) stationär befestigt ist und die Gantry (2) des CT-Geräts mittels einer in der Aufstandsfläche verlaufenden Linearführung (4) relativ zur Plattform (1) verfahrbar ist, und die Plattform (1) als Tragwerk mit parallel zur Aufstandsfläche verlaufenden Trägern (6) ausgeführt ist, das in seitlich des Tragwerks angeordneten Rahmenteilen (5a, 5b) heb- und senkbar gelagert ist. Mithilfe der Erfindung wird somit eine Vorrichtung zur veterinärmedizinischen Diagnostik mit einem CT-Gerät bereitgestellt, die in ihrem Aufbau und ihrer Steuerung technisch einfacher und somit kostengünstiger als bekannte Ausführungen umsetzbar ist. Zudem wird der Untersuchungsvorgang für das Tier angenehmer gestaltet.

## Beschreibung

Die Erfindung betrifft eine Vorrichtung zur veterinärmedizinischen Diagnostik mit einem eine Gantry umfassenden Computertomographie (CT)-Gerät und einem die Öffnung der Gantry querenden CT-Tisch, gemäß dem Oberbegriff von Anspruch 1.

In der veterinärmedizinischen Diagnostik sind mehrere bildgebende Verfahren bekannt, beispielsweise Röntgenverfahren, Ultraschall-Verfahren, die Magnetresonanztomographie (MRT) oder die Computertomograhie (CT), die je nach der zu untersuchenden Körperregion oder medizinischen Verdachtslage Anwendung finden. Während sich beispielsweise Ultraschall gut zur Diagnostik an Sehnen, Bändern der Gelenkkapseln als auch an Herz oder Lunge und im gynäkologischen Bereich eignet, wird die Computertomographie mit großem Erfolg zur Diagnose akuter oder chronischer Lahmheiten, von Kopferkrankungen, Zahnproblemen oder spinaler Ataxie eingesetzt. Bei Verdacht auf Knochenzysten und Fissuren, die auf konventionellen Röntgenbildern nicht gesehen werden können, ist die Computertomografie im veterinärmedizinischen Bereich unverzichtbar.

Ein Computertomograph besteht aus einem Röntgenstrahler und einem gegenüberliegenden Detektor. Diese Komponenten sind innerhalb einer ring- oder torusförmigen Umhüllung angeordnet, die als "Gantry" bezeichnet wird. Die Gantry weist eine zentrale Öffnung auf, die vom CT-Tisch gequert wird, und innerhalb derer sich die zu untersuchende Körperregion des Tieres befinden muss. Der Röntgenstrahler und der Detektor rotieren während der Untersuchung um den CT-Tisch. Hierbei werden bei jeder Schicht Ansichten aus verschiedenen Richtungen erstellt, aus denen computerunterstützt ein Querschnittsbild erstellt werden kann. Durch Weiterbewegen des CT-Tisches können für aufeinanderfolgende Schichten Querschnittsbilder erstellt werden, aus denen in weiterer Folge 3D-Rekonstruktionen errechnet werden können. Hierbei entstehen im Gegensatz zum konventionellen Röntgen keine Überlagerungen von Weichteil- oder Knochengewebe. Die verschiedenen Gewebearten wie Knochen, Muskeln oder Fettgewebe sind auf dem CT-Bild als Kontraste gut erkennbar. Unterschiedliche Graustufen im computertomographischen Bild entsprechen dabei unterschiedlichen Dichten des Gewebes. Die Dichte einzelner Strukturen kann auch objektiv gemessen werden, wodurch auffällige Befunde analysiert und Gewebe sowie Organe eindeutig identifiziert werden können. Mit Hilfe der computertomographischen Bilder ist die genaue Lokalisation krankhafter Strukturen und damit eine bessere Planung des weiteren therapeutischen oder chirurgischen Vorgehens möglich.

Freilich stellt eine CT-Untersuchung von Tieren aus praktischen Gründen mitunter eine Herausforderung dar, insbesondere bei Großtieren wie Pferden. Um Bewegungsartefakte im rekonstruierten 3D-Bild zu vermeiden, muss das Tier während der Untersuchung möglichst bewegungslos verharren. Eine Möglichkeit besteht in der Narkotisierung des Tieres. Eine Narkose stellt aber nicht nur eine physiologische Belastung des Tiers dar, sondern stellt das Personal auch vor große Herausforderungen aufgrund des hohen Eigengewichts eines Großtieres wie beispielsweise eines Pferdes, wenn das Tier in den Untersuchungsraum gebracht werden muss, auf dem CT-Tisch platziert werden muss und rechtzeitig vor dem Aufwachvorgang aufgrund der oft panikartigen Reaktion eines aus der Narkose aufwachenden Großtieres wieder aus dem Untersuchungsraum gebracht werden muss. Daher wird bei Großtieren in der Regel von einer Narkose Abstand genommen und die Untersuchung am wachen Tier vorgenommen, das in den Untersuchungsraum geführt, dort untersucht und danach wieder aus dem Untersuchungsraum geführt wird. Da beispielsweise Pferde Fluchttiere sind, die insbesondere in den beengten Verhältnissen eines CT-Untersuchungsraumes zu Fluchtreaktionen neigen, wird in der Regel zur Beruhigung des Tieres eine Sedierung verabreicht.

Die Untersuchung selbst wird dabei am sedierten, aber stehenden Tier vorgenommen.

Bei einem Großtier wie einem Pferd kann dabei freilich nicht der gesamte Körper untersucht werden. Die Gantry hat eine Öffnung mit einem Durchmesser von etwa 50-90 cm, sodass es beispielsweise bei einem ausgewachsenen Pferd nicht möglich ist das gesamte Tier zu untersuchen, sondern lediglich einzelne Gliedmaßen des Tieres, die das Pferd auf den CT-Tisch stellt, oder den Kopfbereich des Tieres, indem das Tier den Kopf in die Öffnung der Gantry hält. Hierfür ist es auch bekannt das Tier in einen Stand zu stellen, der sich auf einer Hebebühne befindet, sodass das Pferd auf die Höhe der Gantry heruntergefahren werden kann, sodass es den Kopf bequemer in die Gantry halten kann. Bei einem erwachsenen Pferd können somit die vier Gliedmaßen, der Kopf sowie der Hals bis etwa zum fünften Halswirbel untersucht werden. Bei kleineren Pferden oder Fohlen ist auch eine Untersuchung der gesamten Wirbelsäule möglich.

Im Gegensatz zu Untersuchungen im humanmedizinischen Bereich ist es zudem bei der Computertomographie von Großtieren wie etwa Pferden kaum möglich in herkömmlicher Weise den CT-Tisch durch die Gantry zu bewegen. Stattdessen wird die Gantry relativ zum CT-Tisch und somit relativ zum Tier bewegt. So wird beispielsweise in der gattungsgemäßen EP 3838158 A1 eine Computertomographievorrichtung zum Untersuchen eines Körperteils eines Großtieres beschrieben, die ein CT-Gerät und eine Plattform, auf der das CT-Gerät montiert ist, aufweist. Die Gantry ist dabei relativ zur Plattform unbeweglich. Der Computertomograph umfasst des Weiteren eine Verschiebevorrichtung, die mit der Plattform verbunden und ausgebildet ist, die Plattform mit dem montierten CT-Gerät relativ zu einer Standfläche eines Großtieres zu verschieben. Der CT-Tisch ist dabei relativ zur Plattform und zur Gantry horizontal beweglich mit der Gantry und/oder der Plattform derart verbunden, dass der CT-Tisch in die Gantry so eingefahren werden kann, dass der CT-Tisch während einer Relativbewegung zur Gantry relativ zur Standfläche stationär bleibt.

Die in der EP 3838158 A1 beschriebene Computertomographievorrichtung weist somit wie andere bekannte Vorrichtungen, bei denen das CT-Gerät auf einer horizontal beweglichen Plattform angeordnet wird, den Nachteil auf, dass der CT-Tisch die Horizontalbewegung des CT-Geräts kompensieren muss, um eine Untersuchung am ruhenden Tier zu ermöglichen. Das resultiert in technisch komplexe Lösungen, weil die Bewegung der Plattform und der Gantry mit jener des CT-Tisches koordiniert werden muss. Zudem müssen diese Bewegungsvorgänge auch mit der Steuerung des CT-Vorganges abgestimmt werden, sodass die Steuerung des CT-Geräts mit der Steuerung der Horizontalbewegungen der Plattform verknüpft werden muss. Ein weiteres Problem besteht darin, dass die Plattform mit dem CT-Gerät und dem CT-Tisch ein hohes Eigengewicht aufweisen, das horizontal unter präziser Einstellbarkeit des Verstellweges in Bewegung versetzt, gestoppt und in der Ruhelage arretiert werden muss. Lösungen dieser Art sind somit entsprechend aufwändig und teuer.

Es besteht somit das Ziel der Erfindung darin eine Vorrichtung zur veterinärmedizinischen Diagnostik mit einem CT-Gerät bereit zu stellen, die in ihrem Aufbau und ihrer Steuerung technisch einfacher und somit kostengünstiger umsetzbar ist. Zudem soll die erfindungsgemäße Vorrichtung den Untersuchungsvorgang für das Tier so angenehm wie möglich gestalten.

Dieses Ziel wird durch die Merkmale von Anspruch 1 erreicht. Anspruch 1 bezieht sich auf eine Vorrichtung zur veterinärmedizinischen Diagnostik mit einem eine Gantry umfassenden Computertomographie (CT)-Gerät und einem die Öffnung der Gantry querenden CT-Tisch, bei der erfindungsgemäß vorgeschlagen wird, dass eine eine ebene Aufstandsfläche definierende Plattform vorgesehen ist, die mittels einer Hebe- und Senkvorrichtung mit ihrer Aufstandsfläche heb- und senkbar ist, wobei der CT-Tisch auf der Plattform stationär befestigt ist und die Gantry des CT-Geräts mittels einer in der Aufstandsfläche verlaufenden Linearführung relativ zur Plattform verfahrbar ist, und die Plattform als Tragwerk mit parallel zur Aufstandsfläche verlaufenden Trägern ausgeführt ist, das in seitlich des Tragwerks angeordneten Rahmenteilen mittels der Hebe- und Senkvorrichtung heb- und senkbar gelagert ist. Im Unterschied zu herkömmlichen Vorrichtungen ist somit die Plattform, auf der das CT-Gerät angeordnet wird, nicht horizontal verschiebbar, sondern heb- und senkbar ausgeführt, um die Gantry stufenlos auf unterschiedliche Höhenlagen verfahren zu können. Auf diese Weise kann die Positionierung der Gantry auf die Größe des Tieres abgestimmt werden, um beispielsweise durch entsprechendes Absenken der Gantry einen Huf des Tieres bequem untersuchen zu können, oder durch entsprechendes Anheben der Gantry den Kopf des Tieres auf eine für das Tier stressfreie Weise untersuchen zu können. Das Tier steht dabei auf einer unbewegten Standfläche auf Bodenniveau neben der heb- und senkbaren Plattform. Der CT-Tisch ist auf der Plattform stationär befestigt, sodass er relativ zur Plattform unbewegt bleibt. Das CT-Gerät mit seiner Gantry ist hingegen mittels einer in der Aufstandsfläche verlaufenden Linearführung relativ zur Plattform verfahrbar. Ausführungen dieser Art sind aus der Humanmedizin bekannt, wo sie auch als "sliding gantry" bekannt sind. Während normalerweise der Patient mit dem CT-Tisch durch die Gantry bewegt wird, bewegt sich beim "Sliding-Gantry-CT" das CT-Gerät während der Bildgebung entlang des Patienten, der auf einem unbewegten CT-Tisch liegt. Daraus ergeben sich effizientere Nutzungsmöglichkeiten für die therapeutische Bildgebung, indem es möglich wird einen einzigen Tomographen abwechselnd in zwei Untersuchungsräumen einzusetzen. Das CT-Gerät ist dabei auf einer Linearführung gelagert, beispielsweise auf Schienen, die im Boden verlegt sind und von einem Untersuchungsraum in einen angrenzenden Untersuchungsraum führen, und mit einem entsprechenden Fahrsystem versehen, mit dem das CT-Gerät entlang der Linearführung bewegt wird. Die erfindungsgemäße Vorrichtung ermöglicht somit die Verwendung einer herkömmlichen "sliding gantry", die auf der erfindungsgemäßen Plattform angeordnet wird. Die Steuerung des CT-Geräts muss dabei nicht in die Steuerung der Plattform integriert werden, sodass kein Eingriff in die Steuerung des CT-Geräts oder Abgriff von Steuerungssignalen des CT-Geräts erfolgen muss, was die technische Umsetzung der Kombination von Tomograph und Plattform entscheidend erleichtert. Die Plattform muss lediglich eine sichere und stabile Lagerung des CT-Geräts gewährleisten. Hierfür ist die Plattform als Tragwerk mit parallel zur Aufstandsfläche verlaufenden Trägern ausgeführt, das in seitlich des Tragwerks angeordneten Rahmenteilen heb- und senkbar gelagert ist. Das erfindungsgemäße Tragwerk ermöglicht bei vergleichsweise geringem Eigengewicht biegesteife und tragfähige Ausführungen.

Eine konkrete Ausführung sieht etwa vor, dass die Hebe- und Senkvorrichtung zumindest vier Gewindespindeln umfasst, die in den seitlichen Rahmenteilen des Tragwerks angeordnet sind, sowie zumindest einen in den seitlichen Rahmenteilen des Tragwerks angeordneten Antrieb für die Gewindespindeln, wobei die Plattform mit Spindelmuttern versehen ist, mit denen die Plattform bei Rotation der Gewindespindeln entlang der Gewindespindeln heb- und senkbar ist. Die Gewindespindeln ermöglichen auf technisch einfache Weise eine stufenlose und genaue Einstellbarkeit der vertikalen Stellbewegung. Die Plattform wird in der Regel eine rechteckige Form aufweisen, wobei zwei seitliche Rahmenteile auf gegenüberliegenden Seiten der rechteckigen Plattform angeordnet sind und jeweils zwei Gewindespindeln für jede Seite vorgesehen sind.

Insbesondere können zwei Antriebe vorgesehen sein, die jeweils zwei Gewindespindeln zugeordnet sind, wobei jeder Antrieb über eine Verteilerwelle mit den ihm zugeordneten Gewindespindeln verbunden ist. Auf diese Weise werden zwei Gewindespindeln in ihrer vertikalen Stellbewegung koordiniert. Weist die Plattform etwa rechteckige Form auf, bei der zwei seitliche Rahmenteile auf gegenüberliegenden Seiten der rechteckigen Plattform angeordnet sind, befindet sich auf jeder Seite ein Antrieb für die beiden auf der jeweiligen Seite angeordneten Gewindespindeln.

Zudem kann vorgesehen sein, dass die Plattform an Riemen befestigt ist, die mittels Umlenkrollen in den seitlich des Tragwerks angeordneten Rahmenteilen umlaufend angeordnet sind. Die Riemen decken die in Richtung der bewegten Plattform offen auszuführenden Rahmenteile ab und dienen somit als Einklemmschutz sowie zur Vorbeugung von Verschmutzungen der in den seitlichen Rahmenteilen angeordneten Komponenten der Hebe- und Senkvorrichtung.

Hinsichtlich der Heb- und Senkbewegung der Plattform wird insbesondere vorgeschlagen, dass die Aufstandsfläche in eine oberhalb eines Bodenniveaus liegende Position hebbar und in eine unterhalb des Bodenniveaus liegende Position senkbar ist. Das Tier steht dabei auf einer Standfläche auf Bodenniveau. Durch entsprechendes Anheben der Aufstandsfläche in eine oberhalb eines Bodenniveaus liegende Position kann der Kopf des Tieres auf eine für das Tier stressfreie Weise untersucht werden. Durch Absenken der Aufstandsfläche in eine unterhalb des Bodenniveaus liegende Position kann beispielsweise ein Huf des Tieres bequem untersucht werden. Für ein Absenken der Aufstandsfläche in eine unterhalb des Bodenniveaus liegende Position kann eine Montagegrube im Untersuchungsraum vorgesehen sein, in der die seitlichen Rahmenteile mit der Plattform angeordnet werden und in die die Plattform abgesenkt wird.

Des Weiteren wird vorgeschlagen, dass die Aufstandsfläche im Bereich des CT-Tisches mittels eines an die Plattform koppelbaren und entkoppelbaren Randteiles verkleinerbar ist. Diese Maßnahme ermöglicht es dem Tier näher an den CT-Tisch heranzutreten, um die Untersuchung einfacher bewerkstelligen zu können. Werden das CT-Gerät mit dem CT-Tisch angehoben, kann der Randteil von der Plattform entkoppelt werden, sodass der Randteil nicht angehoben wird und das Tier näher and den CT-Tisch treten kann. Das Koppeln und Entkoppeln kann etwa mithilfe eines Bajonett-Verschlusses umgesetzt werden, wobei wechselweise eine Kopplung mit der Plattform und der Schachtwand einer Montagegrube bewerkstelligt werden kann.

CT-Geräte bedürfen in der Regel einer Kühlung, wobei das Kühlaggregat im Fall wassergekühlter Geräte zumeist oberhalb des CT-Geräts angeordnet ist und mit dem CT-Gerät über Kühlmittelleitungen verbunden ist. Für solche Ausführungen wird vorgeschlagen, dass an der Plattform ein Traggestell für ein Kühlaggregat für das CT-Gerät befestigt ist. Das Kühlaggregat wird somit beim Heben und Senken der Plattform mitbewegt, sodass keine Relativbewegungen zwischen Kühlaggregat und CT-Gerät auftreten, die technisch aufwändig kompensiert werden müssten.

Die Erfindung wird in weiterer Folge anhand eines Ausführungsbeispiels mithilfe der beiliegenden Figuren näher erläutert. Es zeigen hierbei die
Fig. 1 eine perspektivische Ansicht einer Ausführungsform der erfindungsgemäßen Vorrichtung mit CT-Gerät, CT-Tisch und Plattform,
Fig. 2 eine weitere Ansicht der Ausführungsform der erfindungsgemäßen Vorrichtung gemäß Fig. 1,
Fig. 3 eine perspektivische Ansicht einer Ausführungsform der zwischen seitlichen Rahmenteilen heb- und senkbar gehaltenen Plattform für die erfindungsgemäße Vorrichtung der Fig. 1,
Fig. 4 eine perspektivische Ansicht einer Ausführungsform der zwischen seitlichen Rahmenteilen heb- und senkbar gehaltenen Plattform für die erfindungsgemäße Vorrichtung der Fig. 1 mit den Komponenten der Hebe- und Senkvorrichtung, und die
Fig. 5 eine perspektivische Ansicht einer Ausführungsform der zwischen seitlichen Rahmenteilen heb- und senkbar gehaltenen Plattform für die erfindungsgemäße Vorrichtung der Fig. 1 mit CT-Gerät und CT-Tisch.

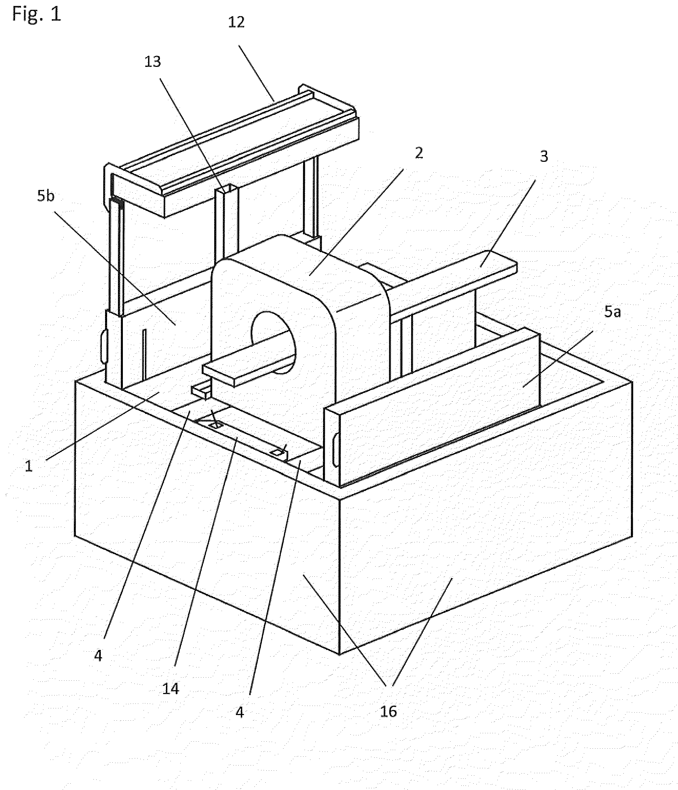

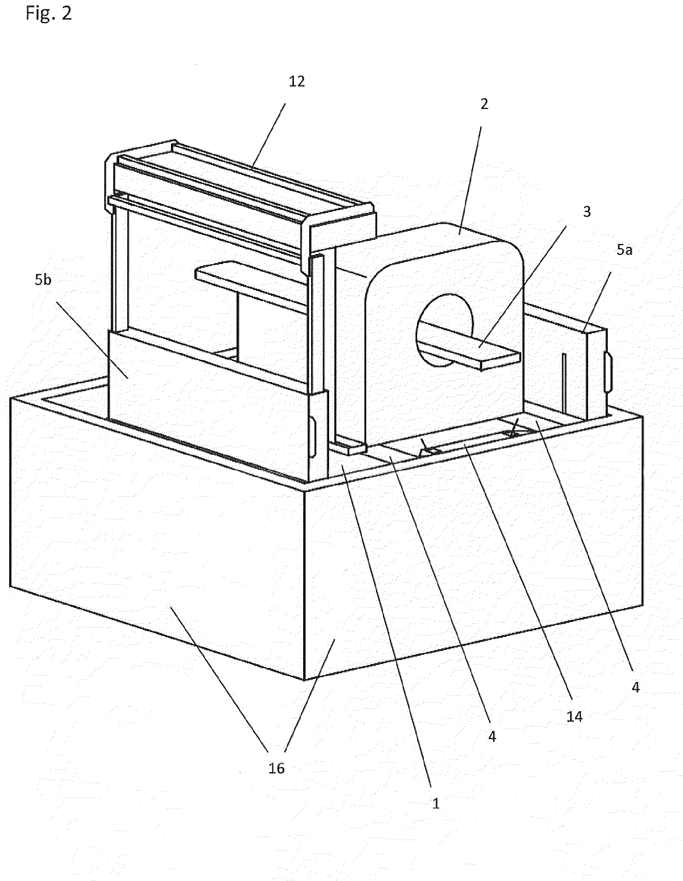

Zunächst wird auf die Fig. 1 und 2 Bezug genommen, die unterschiedliche Ansichten einer Ausführungsform der erfindungsgemäßen Vorrichtung mit einer heb- und senkbaren Plattform 1 sowie einem auf der Plattform 1 angeordneten Computertomographen zeigen. Der Computertomograph umfasst eine ring- oder torusförmige Gantry 2, in der ein Röntgenstrahler und ein gegenüberliegender Detektor angeordnet sind. Die Gantry 2 weist eine zentrale Öffnung auf, die von einem CT-Tisch 3 gequert wird. Der Röntgenstrahler und der Detektor rotieren während der Untersuchung um den CT-Tisch 3.

Die Plattform 1, auf der der Computertomograph angeordnet ist, ist heb- und senkbar ausgeführt, wie noch näher ausgeführt werden wird, um die Gantry 2 stufenlos auf unterschiedliche Höhenlagen verfahren zu können. Auf diese Weise kann die Positionierung der Gantry 2 auf die Größe des Tieres abgestimmt werden, um beispielsweise durch entsprechendes Absenken der Gantry 2 einen Huf des Tieres bequem untersuchen zu können, oder durch entsprechendes Anheben der Gantry 2 den Kopf des Tieres auf eine für das Tier stressfreie Weise untersuchen zu können. Das Tier steht dabei auf einer unbewegten Standfläche auf Bodenniveau neben der heb- und senkbaren Plattform 1, in Bezug auf die Fig. 1 links neben der Plattform 1 im Vordergrund der Fig. 1 und in Bezug auf die Fig. 2 rechts neben der Plattform 1 im Vordergrund der Fig. 2.

Der CT-Tisch 3 ist auf der Plattform 1 stationär befestigt, sodass er relativ zur Plattform 1 unbewegt bleibt und mit der Plattform 1 mitbewegt wird. Das CT-Gerät mit seiner Gantry 2 ist hingegen mittels einer in der Aufstandsfläche der Plattform 1 verlaufenden Linearführung 4 relativ zur Plattform 1 verfahrbar. In der gezeigten Ausführungsform umfasst die Linearführung 4 zwei Schienen, die in der Aufstandsfläche der Plattform 1 verlegt sind. Für die Aufnahme unterschiedlicher Schichtbilder bewegt sich die Gantry 2 in Längsrichtung des unbewegten CT-Tisches 3 mithilfe der Linearführung 4 und eines nicht näher dargestellten Fahrsystems des CT-Geräts entweder schrittweise oder kontinuierlich wie im Falle von Mehrzeilen-Spiral-CTs. CT-Geräte mit Fahrsystemen dieser Art sind wie erwähnt bekannt und werden auch als "sliding gantries" bezeichnet. Auch die Steuerungen dieser CT-Geräte für die Aufnahme unterschiedlicher Schichtbilder können unmittelbar für die erfindungsgemäße Vorrichtung verwendet werden.

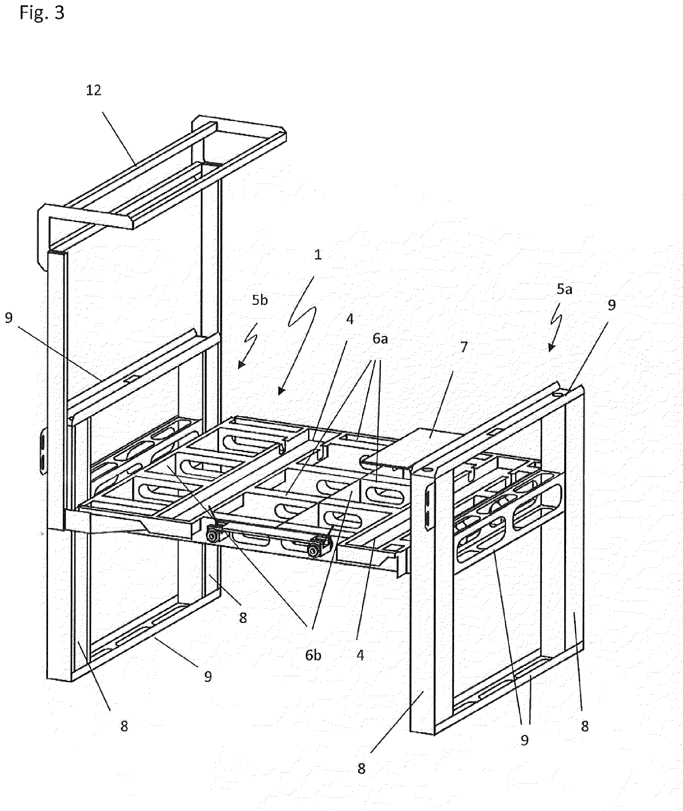

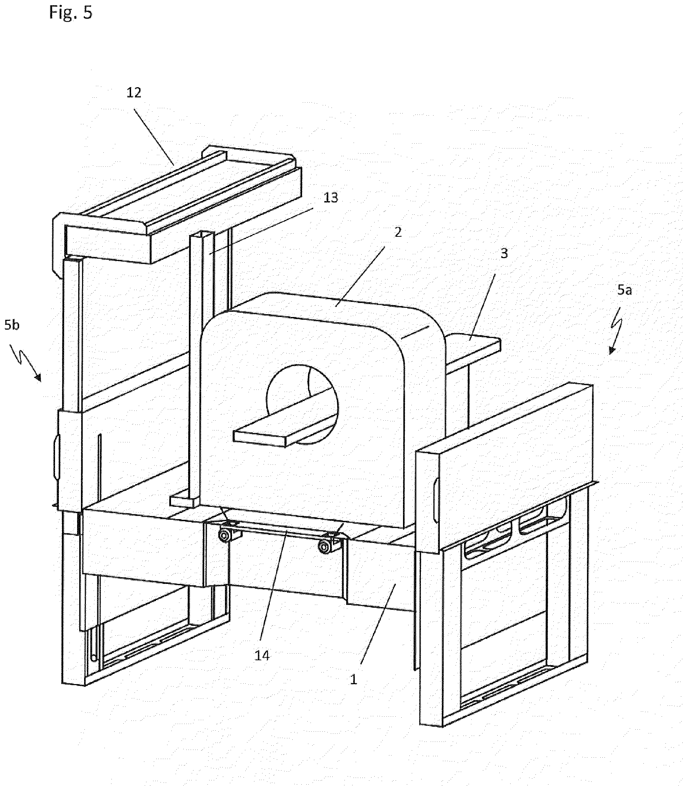

Der Aufbau der Plattform 1 und ihrer seitlichen Rahmenteile 5 wird in weiterer Folge anhand der Fig. 3 bis 5 erläutert. Die Plattform 1 ist als Tragwerk ausgeführt, das in den seitlich des Tragwerks angeordneten Rahmenteilen 5 heb- und senkbar gelagert ist und aus Trägern 6 gebildet wird, die parallel zur Aufstandsfläche der Plattform 1 verlaufen. Im gezeigten Ausführungsbeispiel der Fig. 3-5 verläuft eine erste Schar von Trägern 6a senkrecht zur Laufrichtung der Linearführung 4 für die Gantry 2, und eine zweite Schar von Trägern 6b verläuft parallel zur Laufrichtung der Linearführung 4 und verbindet die erste Schar von Trägern 6a. Der von den jeweils äußersten Trägern 6 gebildete Rahmen der Plattform 1 weist eine im Wesentlichen rechteckige Form auf. Die Träger 6 sind aus einem metallischen Material gefertigt, beispielsweise aus Stahl. Diese Art des Tragwerks stellt eine biegesteife und belastbare Ausführung bei vergleichsweise geringem Eigengewicht dar.

Die Linearführung 4 selbst umfasst im gezeigten Ausführungsbeispiel zwei Schienen, die als wannenförmige Vertiefungen ausgeführt sind, die von entsprechenden Ausnehmungen an der Oberseite der Träger 6 aufgenommen sind. Die Form und Ausführung der Linearführung 4 wird freilich auf den Computertomographen abgestimmt sein und kann je nach verwendetem Modell unterschiedlich sein. An der Oberseite der Träger 6 ist ferner eine Montageplatte 7 für den CT-Tisch 3 befestigt.

Im Ausführungsbeispiel der Fig. 3-5 sind zwei Rahmenteile 5a, 5b an gegenüberliegenden Seiten der rechteckig ausgeführten Plattform 1 angeordnet. Die seitlichen Rahmenteile 5a, 5b werden dabei jeweils aus zwei Stehern 8 und mehreren Querteilen 9, die die Steher 8 verbinden, gebildet. Die Steher 8 weisen jeweils ein U-Profil auf, das sich in Richtung der Plattform 1 öffnet. In den U-Profilen der Steher 8 ist jeweils eine vertikale Gewindespindel drehbar gelagert, sodass insgesamt vier Gewindespindeln vorgesehen sind. Die Plattform 1 ist in ihren vier Eckbereichen mit Vorsprüngen versehen, die in das U-Profil eines dem jeweiligen Eckbereich zugeordneten Stehers 8 eindringen und mit entsprechenden Spindelmuttern versehen sind, die mit der jeweiligen Gewindespindel zusammen wirken.

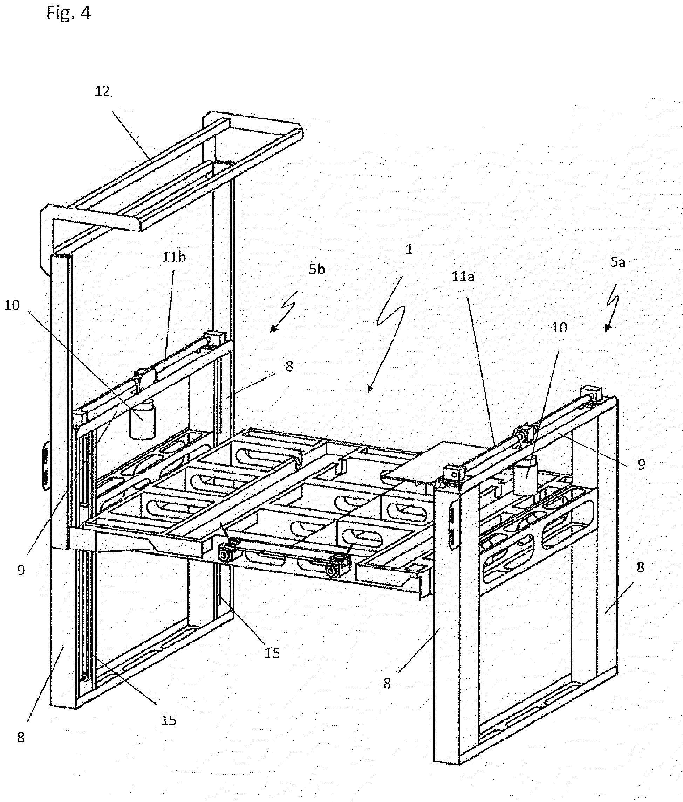

Wie insbesondere der Fig. 4 entnommen werden kann, ist an einem oberen Querteil 9 der Rahmenteile 5a, 5b jeweils ein Antrieb 10 für eine horizontal verlaufende Verteilerwelle 11a, 11b angeordnet. Die Verteilerwelle 11a, 11b überträgt das Drehmoment vom jeweiligen Antrieb 10 auf die oberen Enden der vertikal verlaufenden Gewindespindeln und synchronisiert zudem deren Drehbewegung. Die Hebe- und Senkvorrichtung wird somit im Wesentlichen aus den Antrieben 10, den Verteilerwellen 11a, 11b, den Gewindespindeln und den Spindelmuttern gebildet.

Die Fig. 4 zeigt des Weiteren, dass Riemen 15 innerhalb der Steher 8 vorgesehen sind, die mittels Umlenkrollen in den seitlich des Tragwerks angeordneten Rahmenteilen 5a, 5b umlaufend angeordnet und an der Plattform 1 befestigt sind. Die Riemen 15 decken die in Richtung der bewegten Plattform 1 offen ausgeführten Steher 8 ab und dienen somit als Einklemmschutz sowie zur Vorbeugung von Verschmutzungen der in den seitlichen Rahmenteilen 5a, 5b angeordneten Komponenten der Hebe- und Senkvorrichtung.

An der Plattform 1 ist des Weiteren ein Traggestell 12 für ein Kühlaggregat für das CT-Gerät befestigt. CT-Geräte bedürfen wie erwähnt in der Regel einer Kühlung, wobei das Kühlaggregat im Fall wassergekühlter Geräte zumeist oberhalb des CT-Geräts angeordnet ist und mit dem CT-Gerät über Kühlmittelleitungen 13 verbunden ist. Das Kühlaggregat wird somit beim Heben und Senken der Plattform 1 mitbewegt, sodass keine Relativbewegungen zwischen Kühlaggregat und CT-Gerät auftreten, die technisch aufwändig kompensiert werden müssten.

Im gebrauchsfertigen Zustand sind die Oberseite des Tragwerks der Plattform 1 und die Außenseiten der Rahmenteile 5a, 5b durch Platten abgedeckt, sodass sich die Konfiguration der Fig. 1 und 2 ergibt.

Um eine Absenkbarkeit der Plattform 1 unter das Bodenniveau zu ermöglichen, wird die erfindungsgemäße Vorrichtung in einer Montagegrube mit vertikalen Schachtwänden 16 angeordnet sein, wie den Fig. 1 und 2 ersichtlich ist. Die seitlichen Rahmenteile 5a, 5b können an den Schachtwänden 16 befestigt werden und überragen in der gezeigten Ausführungsform der Fig. 1 und 2 die Montagegrube und somit das Bodenniveau.

Des Weiteren ist die Aufstandsfläche im Bereich des CT-Tisches 3 mittels eines an die Plattform 1 koppelbaren und entkoppelbaren Randteiles 14 verkleinerbar. Diese Maßnahme ermöglicht es dem Tier näher an den CT-Tisch 3 heranzutreten, um die Untersuchung einfacher bewerkstelligen zu können. Werden die Plattform 1 mit dem CT-Gerät und dem CT-Tisch 3 angehoben, kann der Randteil 14 von der Plattform 1 entkoppelt werden, sodass der Randteil 14 nicht angehoben wird und das Tier näher and den CT-Tisch 3 treten kann. Das Koppeln und Entkoppeln kann etwa mithilfe eines Bajonett-Verschlusses umgesetzt werden, wobei wechselweise eine Kopplung mit der Plattform 1 und der Schachtwand 16 der Montagegrube bewerkstelligt werden kann.

Im Unterschied zu herkömmlichen Vorrichtungen ist somit die Plattform 1, auf der das CT-Gerät angeordnet wird, nicht horizontal verschiebbar, sondern heb- und senkbar ausgeführt, um die Gantry 2 stufenlos auf unterschiedliche Höhenlagen verfahren zu können. Der CT-Tisch ist auf der Plattform 1 stationär befestigt, sodass er relativ zur Plattform 1 unbewegt bleibt. Das CT-Gerät ist hingegen mittels der in der Aufstandsfläche verlaufenden Linearführung 4 relativ zur Plattform 1 verfahrbar. Die erfindungsgemäße Vorrichtung ermöglicht somit die Verwendung einer herkömmlichen "sliding gantry", die auf der erfindungsgemäßen Plattform 1 angeordnet wird. Die Steuerung des CT-Geräts muss dabei nicht in die Steuerung der Plattform 1 integriert werden, sodass kein Eingriff in die Steuerung des CT-Geräts oder Abgriff von Steuerungssignalen des CT-Geräts erfolgen muss, was die technische Umsetzung der Kombination von Tomograph und Plattform 1 entscheidend erleichtert.

Mithilfe der Erfindung wird somit eine Vorrichtung zur veterinärmedizinischen Diagnostik mit einem CT-Gerät bereitgestellt, die in ihrem Aufbau und ihrer Steuerung technisch einfacher und somit kostengünstiger als bekannte Ausführungen umsetzbar ist. Zudem wird der Untersuchungsvorgang für das Tier so angenehm wie möglich gestaltet.

## Patentansprüche

1. Vorrichtung zur veterinärmedizinischen Diagnostik mit einem eine Gantry (2) umfassenden Computertomographie (CT)-Gerät und einem die Öffnung der Gantry (2) querenden CT-Tisch (3), **dadurch gekennzeichnet, dass** eine eine ebene Aufstandsfläche definierende Plattform (1) vorgesehen ist, die mittels einer Hebe- und Senkvorrichtung mit ihrer Aufstandsfläche heb- und senkbar ist, wobei der CT-Tisch (3) auf der Plattform (1) stationär befestigt ist und die Gantry (2) des CT-Geräts mittels einer in der Aufstandsfläche verlaufenden Linearführung (4) relativ zur Plattform (1) verfahrbar ist, und die Plattform (1) als Tragwerk mit parallel zur Aufstandsfläche verlaufenden Trägern (6) ausgeführt ist, das in seitlich des Tragwerks angeordneten Rahmenteilen (5a, 5b) mittels der Hebe- und Senkvorrichtung heb- und senkbar gelagert ist.

2. Vorrichtung nach Anspruch 1, **dadurch gekennzeichnet, dass** die Hebe- und Senkvorrichtung zumindest vier Gewindespindeln umfasst, die in den seitlichen Rahmenteilen (5a, 5b) des Tragwerks angeordnet sind, sowie zumindest einen in den seitlichen Rahmenteilen (5a, 5b) des Tragwerks angeordneten Antrieb (10) für die Gewindespindeln, wobei die Plattform (1) mit Spindelmuttern versehen ist, mit denen die Plattform (1) bei Rotation der Gewindespindeln entlang der Gewindespindeln heb- und senkbar ist.

3. Vorrichtung nach Anspruch 2, **dadurch gekennzeichnet, dass** zwei Antriebe (10) vorgesehen sind, die jeweils zwei Gewindespindeln zugeordnet sind, wobei jeder Antrieb (10) über eine Verteilerwelle (11a, 11b) mit den ihm zugeordneten Gewindespindeln verbunden ist.

4. Vorrichtung nach einem der Ansprüche 1 bis 3, **dadurch gekennzeichnet, dass** die Plattform (1) an Riemen (15) befestigt ist, die mittels Umlenkrollen in den seitlich des Tragwerks angeordneten Rahmenteilen (5a, 5b) umlaufend angeordnet sind.

5. Vorrichtung nach einem der Ansprüche 1 bis 4, **dadurch gekennzeichnet, dass** die Aufstandsfläche in eine oberhalb eines Bodenniveaus liegende Position hebbar und in eine unterhalb des Bodenniveaus liegende Position senkbar ist.

6. Vorrichtung nach einem der Ansprüche 1 bis 5, **dadurch gekennzeichnet, dass** die Aufstandsfläche im Bereich des CT-Tisches (3) mittels eines an die Plattform (1) koppelbaren und entkoppelbaren Randteiles (14) verkleinerbar ist.

7. Vorrichtung nach einem der Ansprüche 1 bis 6, **dadurch gekennzeichnet, dass** an der Plattform (1) ein Traggestell (12) für ein Kühlaggregat für das CT-Gerät befestigt ist.
